Europäisches Patentamt

European Patent Office

Office européen des brevets

(19)

(11)  EP 0 996 401 B1

(12)  **EUROPEAN PATENT SPECIFICATION**

(45)  Date of publication and mention
of the grant of the patent:
**22.01.2003   Bulletin 2003/04**

(21)  Application number: **98938038.1**

(22)  Date of filing: **23.07.1998**

(51)  Int Cl.[7]: **A61F 13/15**

(86)  International application number:
**PCT/US98/15569**

(87)  International publication number:
**WO 99/004741 (04.02.1999 Gazette 1999/05)**

(54)  **ABSORBENT ARTICLES HAVING A LIQUID SWELLABLE BREATHABLE BACKSHEET**

ABSORBIERENDER ARTIKEL MIT EINER FLÜSSIGKEITSSCHWELLBAREN, ATMUNGSFÄHIGEN, ÄUSSEREN SCHICHT

ARTICLES ABSORBANTS COMPORTANT UNE FEUILLE INFERIEURE RESPIRANTE POUVANT GONFLER AU CONTACT DE LIQUIDES

(84)  Designated Contracting States:
**BE DE ES FR GB GR IT NL SE**

(30)  Priority:  **25.07.1997  EP 97112814**

(43)  Date of publication of application:
**03.05.2000   Bulletin 2000/18**

(73)  Proprietor: **THE PROCTER & GAMBLE COMPANY**
**Cincinnati, Ohio 45202 (US)**

(72)  Inventors:
• **CIAMMAICHELLA, Fabio**
**I-65126 Pescara (IT)**
• **DIVO, Michael**
**D-61381 Friedrichsdorf (DE)**

• **CORZANI, Italo**
**I-66100 Chieti (IT)**

(74)  Representative: **Veronese, Pancrazio et al**
**Procter & Gamble Italia S.p.A.**
**Italian Research Center**
**Via Aterno 92/94**
**66020 Sambuceto di San Giovanni Teatino**
**(Chieti) (IT)**

(56)  References cited:
**EP-A- 0 401 189         EP-A- 0 710 472**
**EP-A- 0 780 108         WO-A-97/15258**
**US-A- 4 235 237         US-A- 4 592 751**
**US-A- 5 580 910**

## Description

Field of the Invention

The present invention relates to the provision of breathable backsheets for use in absorbent articles which are activated when contacted with liquids and thereby prevent garment wet through.

Background of the Invention

The primary consumer needs which underlie development in the absorbent article field, in particular catamenials, is the provision of products providing both a high protection and comfort level.

One means for providing consumer comfort benefits in absorbent articles is by the provision of breathable products. Breathability has typically concentrated on the incorporation of so called 'breathable backsheets' in the absorbent articles. Commonly utilised breathable backsheets are microporous films and apertured formed films having directional fluid transfer as disclosed in for example US 4 591 523. Both these types of breathable backsheets are vapour permeable allowing gaseous exchange with the environment. This thereby allows for the evaporation of a portion of the fluid stored in the core and increases the circulation of air within the absorbent article. The latter is particularly beneficial as it reduces the sticky feeling experienced by many wearers during use, commonly associated with the presence of an apertured formed film or film like topsheet, particularly over extended periods of time. This is a result of topsheets designed to achieve a clean and dry appearance. These topsheets tend to be smooth thereby minimising the build up of fluid on the surface of the topsheet. However, these benefits are achieved at the expense of comfort, particularly under hot and humid conditions, when due to their smooth surface texture they tend to become sticky to the skin.

However, the main drawback associated with the use of breathable backsheets in absorbent articles is the negative effect on the protection level performance, by leakage known as wet through onto the users garment. Although, breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the product is utilised during physical exertion, or for heavy discharge loads or over extended periods of time. Thus, whilst the incorporation of breathable backsheets in absorbent articles is highly desirable from a comfort standpoint, since the primary role of a backsheet still remains the prevention of liquid leakage, such breathable backsheets cannot be satisfactorily incorporated into products.

The problem of wet through onto users garments due to the incorporation of such breathable backsheets in absorbent articles has indeed also been recognised in the art. Attempts to solve the problem have mainly resided in the use of multiple layer backsheets such as those illustrated in US 4 341 216. Similarly EPO 710 471 discloses a breathable backsheet comprising an outer layer of a gas permeable, hydrophobic, polymeric fibrous fabric and an inner layer comprising an apertured formed film having directional fluid transport. The backsheet construction preferably has no liquid transport or wet through under certain specified test conditions. Also EPO 710 472 discloses a breathable backsheet consisting of at least two breathable layers which are unattached to one another over the core area. The backsheet construction preferably has no liquid transport/wet through under certain specified test conditions.

US 4 713 068 discloses a breathable clothlike barrier for use as an outer cover for absorbent articles. The barrier comprises at least 2 layers, a first layer having a specified basis weight, fibre diameter and pore size and a second layer comprising a continuous film of polyvinyl alcohol having a specified thickness. The barrier also has a specified water vapour transmission rate and level of impermeability.

However, none of the above proposed solutions have been able to provide a fully satisfactory solution to the problem of breathable backsheet wet through under all conditions. Furthermore, another problem associated with the exemplified multi layer backsheets is an increase in total thickness of the product and a reduction in the flexibility, both of which result in a consumer noticeable reduction in product comfort.

An alternative proposed solution to the problem of breathable backsheet wet through relates to the improvement of the absorbent material such that little or no liquid comes into contact with the backsheet, thereby preventing wet through. This is typically achieved by increasing the amount of absorbent material in the article. However, this results in an absorbent article which is extremely thick which is highly undesirable from a consumer comfort standpoint. Hence, the absorbent article whilst having the required protection level and still maintaining some comfort benefits by the presence of the breathable backsheet, suffers from a lack of comfort from a different source, in this case the increased dimensions of the article.

In addition the above solution also results in a reduction in the flexibility of the article, particularly evident as an increase in the cross section stiffness. It is however also well established that in order to be comfortable for the wearer absorbent articles need to be cross sectionally flexible. It is believed that the more cross sectionally flexible an absorbent article is, the less will it be noticeable to the wearer. Thus flexibility is another highly desirable comfort requirement of modern absorbent articles.

EPO 705 583 and EPO 705 584 propose longitudinally flexible absorbent articles which are vapour permeable. However, the exemplified absorbent articles are typically very thin and do not address the absorbency capacity of the article or the problem of wet through.

US 5 447 788 discloses a porous nonwoven liquid activated barrier suitable for use in absorbent articles. The barrier includes a fibrous nonwoven web in which at least 50% of the fibres are prepared from a liquid swellable polymer which is not significantly soluble in water such as swellable polyvinyl alcohols. In the presence of water the polymers swell to an extent sufficient to substantially block the passage of liquid through the fibrous nonwoven web. However, from the data given in the patent, the passage of liquids is not substantially blocked and thus these webs do not eliminate the wet through problem.

US 4 235 237 discloses an absorbent open network having water-insoluble but water-swellable particles spaced from one another but integral with network. This absorbent structure in the swallen condition is substantially impermeable to the flow of liquids.

Consequently, as the incorporation of breathable backsheets in absorbent articles results in reduction of the protection level, further desirable product comfort modifications such as reducing the thickness of the product and improving the flexibility of the product which would further acerbate the problem, may not be incorporated in the absorbent article. Thus, there exists a dichotomy in the means available to provide increased consumer comfort in absorbent products and acceptable protection levels.

It is therefore an objective of the present invention to provide an absorbent article having improved comfort, by the provision of breathability throughout the absorbent article which continues to maintain an acceptable level of protection.

It has now been found that this objective may be achieved by the provision of an article having a breathable backsheet which comprises at least one layer selected from wovens or apertured films comprising a non soluble, liquid swellable material.

An advantage of the present invention is that the backsheet when in the dry state allows the transfer of moisture vapour and preferably both moisture vapour and air and, in its wet state after swellage maintains at least a degree of vapour permeability whilst preventing the transfer of liquids.

## Summary of the Invention

The present invention relates to absorbent articles such as sanitary napkins and panty liners comprising a liquid permeable topsheet, a backsheet and an absorbent core positioned between the topsheet and core, which are breathable by the incorporation of a breathable backsheet and, which have a reduced tendency to exhibit garment wet through. The backsheet comprises at least one vapour permeable layer, selected from wovens or apertured films comprising from 10% to 100% by weight of said layer of a non soluble, liquid swellable material such as polyvinyl alcohol. Upon contact with the liquid discharge the material swells thereby causing the apertures of the layer to become smaller and preferably close. In this manner the passage of liquid through the backsheet onto the garment of the wearer is prevented.

## Detailed Description of the Invention

The absorbent article according to the present invention comprises as an essential component, a breathable backsheet. The backsheet typically extends across the whole of the absorbent structure and can extend into and form part of or all sideflaps, side wrapping elements or wings. The backsheet primarily prevents the extrudes absorbed and contained in the absorbent structure from wetting articles that contact the absorbent product such as underpants, pants, pyjamas and undergarments thereby acting as a barrier to fluid transport. In addition however, the breathable backsheet of the present invention when dry permits the transfer of at least water vapour and preferably both water vapour and air through it and thus allows the circulation of gases into and out of the backsheet. In addition however the backsheet also preferably continues to allow the transfer of water vapour through it after wetting.

According to the present invention the breathable backsheet comprises at least one layer selected from wovens or apertured films. Said layer comprises from 10% to 100%, preferably from 25% to 100%, more preferably from 50% to 100%, most preferably from 75% to 100% by weight of said layer of said backsheet of a non water soluble, liquid swellable material.

Surprisingly, it has been found that the incorporation of the material in a woven layer or an apertured film layer allows the material to swell to such an extent that once the layer is contacted with liquid, the liquid passage through the layer is substantially hindered. Whilst not being bound by theory it appears that the combination of the incorporation of swellable materials in a woven layer or apertured film layer is advantageous because the apertures are distributed in a highly regular manner throughout such layers. In particular it is believed that the highly regular dimensions, geometrical shape and regular and close distribution of the apertures of such layers allows the layer to contract more efficiently, thereby permitting the apertures to close. As a result the liquid penetration through the layer is prevented. This is in contrast to the situation when the swellable material is provided in a nonwoven, where the fibres are in a random configuration. As a result of the random fibre orientation, the apertures distribution as well as the apertures size distribution are also randomly distributed and hence apertures closure is not complete.

According to the present invention any known material which swells when in contact with liquid is suitable for incorporation in the apertured backsheet layer. The term "swell" as used herein refers to a material exhibiting expansion when in contact with liquid in at least one direction i.e. in the x transverse direction, the y longitudinal direction or the z vertical direction or a material which swells in any combination of said directions.

The term liquid as used herein refers to any fluid containing at least 75% by weight of water such as body fluids including urine, menstrual fluid and the like. The term non soluble as used herein refers to a material which is not significantly soluble at body temperature such that it can be utilised for the desired function in absorbent articles.

According to the present invention each backsheet layer or layers comprising the swellable material, swells in at least the x, y, or z direction. Typically, most suitable materials are isotropic such that they swell in all 3 dimensions. According to the test methods described hereafter said material swells by at least 100%, preferably at least 200%, in the z direction of said apertured layer of said backsheet. However, swellage in the x and y directions is also desirable.

The incorporation of at least one woven or apertured film layer comprising a swellable material in the breathable backsheet allows the passage of water vapour and preferably both water vapour and air through it. Thus, before use and prior to contact with liquid and whilst the backsheet remains dry, the absorbent article is always at least permeable to water vapour. After contact with liquid, the material swells thereby tending to close the apertures of said layer of the backsheet. This thereby prevents the passage of macroscopic matter and liquids through the backsheet and onto the garments of the wearer. As a result of the swellage of the layer the air permeability of the layer and hence of the backsheet overall is reduced. Typically the air permeability is negligible once the layer has become wet, which is indicative of the closure of the apertures. An advantage of the present invention however is that the passage of water vapour is not significantly hindered by the swelling action of the material on contact with liquids. Hence whilst the air permeability is reduced the backsheet and consequently the absorbent article still remains breathable even after contact with liquids. In addition the structural integrity of the layer of the backsheet should be maintained after contact with liquid. Obviously, the areas of the layer which do not come into contact with liquid during use of the absorbent article will maintain air and moisture vapour permeability throughout. Hence, the layer is air and moisture vapour permeable not only whilst dry but also retains its air and moisture vapour permeability wherever liquid contact with the layer does not occur.

Preferred breathable layers for use as backsheets herein are those having a high vapour and air exchange when dry and which preferably maintain a degree of vapour permeability once wet, whilst preventing air exchange. Hence, according to the present invention each layer of the backsheet comprising the swellable material has a vapour permeability when dry of at least 500 g/(m$^2$.24hrs), preferably at least 600 g/(m$^2$.24hrs). According to a preferred embodiment of the present invention each swellable layer also has an air permeability when dry of at least 50 l/(m$^2$s), preferably at least 70 l/(m$^2$s). Less preferably when said layer comprising the swellable material is in the form of a 2-dimensional microporous film, in the dry state this backsheet layer is only water vapour permeable. Furthermore, each swellable layer has a air permeability when wet as described herein after under test methods of less than 10 l/(m$^2$s), preferably less than 8 l/(m$^2$s). Furthermore, each swellable layer has a vapour permeability after wetting as described in the test methods of more than 250 g/(m$^2$.24hrs), preferably more than 350 g/(m$^2$.24hrs).

According to the present invention suitable swellable materials for use herein include polymers such as polyvinyl alcohols having a hydrolysis level of at least 85 %, preferably at least 95 %, most preferably at least 98 %. The polyvinyl alcohols are prepared by hydrolysis of polyvinyl acetate. The term hydrolysis level as used herein thus refers to the percent, in moles, of substitution of the acetate groups by hydroxyl groups.

Other suitable materials exhibiting an ability to swell in contact with liquids for use herein include (as non limitative examples) polymers such as crosslinked acrylic acid and its copolymers, especially in the form of full or partial salts of alkaline metals, alkaline-earth metals as well as aluminium, zinc and iron salts, as well as such as poly(methacrylic acid), polyacrylamide, poly(N,N-dimethylacrylamide), and polymethylacrylamide;

crosslinked poly-vinyl-pyrrolidone; copolymers of poly-vinyl-pyrrolidone, particularly those containing vinyl acetate; polymethyl-, polyethyl- or polybutylvinyl ethers as well as the copolymers derived from vinyl ethers as, for example, the copolymers of methyl-vinyl-ether and maleic anhydride or maleic acid;

polyethylene oxide having molecular weights from about 100,000 to 8,000,000 Daltons;

polysaccharides (gums) of natural origin and their semisynthetic derivatives such as alginic acid and its salts, agar, locust bean gum, carrageenans, gelatin, starch and cellulose derivatives such as carboxy-methyl-cellulose and cellulose acetate, pectin, guar gum and xanthan gum; and

polyethylenimine; polyacrolein; styrene-maleic anhydride copolymers; ethylene-maleic anhydride copolymers;

polydimethylaminoethyl methacrylate; polyalkylene polyamines; poly(vinylbenzyl-trimethylammonium chloride) as well similar quaternary ammonium polymers; poly(maleic anhydride); lower molecular weight phenol-formaldehyde resins; lower molecular weight urea-formaldehyde resins, as well as compatible blends of said swellable materials.

According to the present invention said layer may comprise a mixture of swellable materials, which may exhibit different levels of swellability with liquid, in addition to other non swellable components. The swellable material however should be present at least 10% by weight of the layer and the swellability of said layer should remain within the previously specified limits. Hence, said layer of said breathable backsheet may further comprise up to 90% by weight, preferably up to 75 %, more preferably up to 50 % and most preferably up to 25 % by weight, of said layer of non swellable component materials. However in the most preferred embodiment of the present invention, the backsheet layer comprises 100% by weight of said layer of the swellable material.

In such embodiments wherein said layer comprises other components in addition to the swellable materal, the swellable material itself may be distributed throughout the layer in an ordered or random manner. Alternatively, the swellable material may be distributed in discrete regions throughout the layer. Preferably, said swellable material is distributed homogeneously throughout the layer, more preferably in a regular repeating pattern, such as in longitudinal strips throughout the layer.

Suitable non swellable component materials for use herein may be both naturally and synthetically derived and are selected depending on the form in which the layer is to be provided. For example, embodiments wherein the swellable backsheet layer is provided in the form of a fibrous woven, said layer may include non swellable component materials such as e.g. nylon, polyester, cotton and other natural, synthetic or artificial fibrous components. These materials may be intimately mixed within the fibre of the swellable material itself and/or woven together with the swellable material. For embodiments wherein the swellable backsheet layer is in the form of an apertured film, suitable non swellable components are polymers which are compatible with the swellable polymer. For example a swellable layer comprising poly-ethylene oxide as the swellable material, may in addition comprise poly-caprolactone. In addition traditional components of polymeric films such as plasticisers and other usual minor components of these films such as stabilisers (antioxidants, anti-UV), pigments, and mineral fillers may also be incorporated in to the layer. For example for embodiments comprising poly-vinyl alcohol as the swellable material suitable plasticisers include glycerol, glycols, polyglycols, trimethylol propane and triethanolamine.

According to the present invention the breathable backsheet comprises at least one layer comprising said swellable material. Each layer comprising the swellable material such as polyvinyl alcohol, can be provided in forms selected from fibrous wovens, 2-dimensional microporous films, 2-Dimensional macroporous films, macroscopically expanded films, formed apertured films or any combination thereof. Preferably, at least one and more preferably all of the swellable apertured layers of the backsheet are selected from 2-dimensional microporous films or macroporous apertured films or a combination thereof. The construction of these layers themselves are described in more detail herein after under the description relating to the additional or optional backsheet layers which are non liquid swellable .

According to one embodiment of the present invention, the swellable material may be formed into fibres prior to the production of a fibrous woven layer. Typically, said swellable material and optional non swellable materials in the fibrous form, have an average fibre diameter of from 5 micrometers to 300 micrometers, preferably from 10 micrometers to 250 micrometers, most preferably from 50 micrometers to 150 micrometers. The fibre preferably comprises at least 50% by weight, preferably at least 75% by weight, more preferably at least 90% by weight of the fibre of said swellable material.

According to one embodiment of the present invention wherein said liquid swellable material is incorporated into an apertured formed film or a 2-dimensional porous film, the average pore diameter of such layers is preferably from 0.5 micrometers to 1000 micrometers. The size of the apertures will vary however depending on the type of layer utilised.

According to the present invention said backsheet comprises at least one layer comprising said non soluble liquid swellable material. In addition, said backsheet may comprise optional additional layers which are not liquid swellable and hence do not contain swellable materials as defined herein. According to the present invention, said backsheet preferably comprises in total at least two layers and most preferably said backsheet consists of three layers. The additional backsheet layers can be of any form such as polymeric wovens, nonwovens or apertured films such as 2-Dimensional, planar micro and macro-porous films, macroscopically expanded films and formed polymeric apertured films. Furthermore, said additional layers may be of any chemical nature, as described herein after and may be formed from synthetic or naturally derived sources or mixtures thereof.

According to the present invention suitable additional non swellable layers for use in the breathable backsheets herein are layers which are moisture vapour permeable and more preferably both moisture vapour and air permeable. Suitable layers may be fibrous wovens, fibrous nonwovens, apertured films or any mixtures or combinations thereof. Suitable vapour permeable layers include 2-Dimensional, planar micro and macro-porous films, macroscopically expanded films and formed apertured films. According to the present invention the apertures in said layer may be of any configuration, but are preferably spherical or oblong and may also be of varying dimensions. Typically, the apertures have an average diameter of from 5 micrometers to 600 micrometers. 2 dimensional planar porous films for use herein may have apertures having diameters from 200 micrometers to 5 micrometers. 2 dimensional planar microporous layers have apertures having average diameters of from 150 micrometers to 5 micrometers, preferably from 120 micrometers to 10 micrometers, most preferably from 90 micrometers to 15 micrometers. 2 dimensional planar macro porous layers have apertures having an average diameter of from 90 micrometers to 200 micrometers. Macroscopically expanded film layers and formed apertured layers have apertures having an average diameter of from 75 micrometers to 600 micrometers. However, for backsheet layers comprising the swellable material, the average diameter of the apertures in the layer, as well as their distribution throughout the layer, which thereby defines the open area of the layer is selected according to the particular swellable material utilized and its ability to swell and the quantity and distribution of the material in the layer. Hence, in order to obtain high air permeability levels whilst dry, the open area of apertures in the layer should be as large as possible, whilst ensuring that the air permeability will be significantly reduced on contact with liquid. The apertures are therefore preferably evenly distributed across the entire surface of the layer, however layers having only certain regions of the surface having apertures are also envisioned.

Suitable 2-Dimensional planar layers of the backsheet may be made of any material known in the art, but are preferably manufactured from commonly available polymeric materials. Suitable materials are for example XMP-1001 of Minnesota Mining and Manufacturing Company, St. Paul, Minnesota, USA and Exxaire XBF-101W, supplied by the Exxon Chemical Company. As used herein the term 2-Dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein.

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core.

Suitable macroscopically expanded films for use herein include films as described in for example in US 4 637 819 and US 4 591 523.

Preferably, said swellable material is incorporated into at least one of the backsheet layers. In multiple breathable backsheet embodiments wherein only one of the backsheet layers comprises the swellable material of the present invention, this layer should be positioned towards the absorbent core, preferably such that said layer does not form the garment facing surface of the absorbent article. Most preferably said layer is positioned such that it is in direct contact with the absorbent core.

According to a preferred embodiment of the present invention the backsheet comprises at least two layers, a first layer comprising said swellable material and a second layer comprising a non swellable polymeric layer comprising a fibrous woven, a fibrous nonwoven, a 2-Dimensional apertured porous film or a formed apertured film.

According to a most preferred embodiment of the present invention the backsheet consists of three layers, a first layer comprising said non soluble, liquid swellable material and a second and a third layer both comprising non swellable components, wherein the second layer comprises a polymeric apertured formed film and the third layer comprises a polymeric fibrous nonwoven.

According to a preferred embodiment of the present invention the breathable backsheet preferably has in the dry state, both air and water vapour permeability at the levels defined herein above for the swellable layers. More preferably, the breathable backsheet also meets the vapour permeability levels in the wet state.

According to the present invention the absorbent articles further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

Another component which may be included in the absorbent articles of the present invention, in particular in the core are odour control actives. Suitable actives include zeolites, silica, chelants such as ethylene diamine tetraacetic acid, active carbon and clays. These components can be in corporated in any form, but preferably as discrete particles.

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearer's skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

According to the present invention the absorbent article is constructed by joining the various elements such as topsheet, backsheet and absorbent core by any means well known in the art. For example the backsheet and/or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof. Preferably the individual layers of the breathable backsheet are joined to each other so as to minimise and preferably eliminate any reduction in the vapour permeability of the backsheet . Similarly, the breathable backsheet itself is joined to other elements of the absorbent articles so as to minimise the effect of the moisture vapour permeability of the backsheet.

According to the present invention the absorbent article may find utility as sanitary napkins, panty liners, adult incontinence products and baby diapers but may also include other articles such as underarm sweat pads or collar bands. The present invention finds particular susceptibility as sanitary napkins and panty liners. Thus, in addition to the components described herein above, the absorbent article may also comprise all those features and parts which are typical for products in the context of their intended use such as wings and side flaps, undergarment adhesive means, release paper, wrapping elements, fastening means and the like, all well known in the art.

Examples

Swellable backsheet layer examples:

Liquid swellable apertured films, for use in a breathable backsheet construction according to the present invention, were prepared from the following commercially available polyvinyl alcohol films.

Example 1:

"Aquafilm hot water" available from Aquafilm Ltd , U.K.

Example 2:

"Ekosol tipo C 60 °C", available from Ekomer Srl, Italy.

Example 3:

"Ekosol tipo C 80 °C", available from Ekomer Srl, Italy.

The above films were perforated using a steel needled perforator. The perforator consists of steel needles having rectangular dimensions of 530 micrometers by 350 micrometers and a height of 4mm. The steel needles are arranged in a grid formation such that the distance between the centre of any two adjacent rectangular bases of the needle is 5mm.

Dry and wet samples

The term dry as used herein refers to a sample material stored at 23°C and 50% relative humidity for at least 12 hours. The test is also carried out under these conditions.

The term wet as used herein refers to a sample material which has been uniformly contacted with 10 cm$^3$ of distilled water at 37°C with a pipette. The excess water is removed by gently pressing the samples on blotting paper. The sample is left to stand for at least 60 seconds before the test is carried out, the test being carried out within 10 minutes after contacting with water.

Air Permeability test on individual swellable layers of a breathable backsheet.

The air permeability test is utilised to assess the ability of a material to circulation/exchange of air.

Basic Principle of the Method:

The basic principle of the test is to evaluate the resistance of a material to the passage of air. This test measures the volume (or amount) of air that flows through a sample of given dimensions under standard conditions (of 23 °C /50% RH) and under a given difference of pressure. The instrument utilised for the test is: Air Permeabilimeter FX 3300 manufactured by TexTest AG Switzerland.

100 mm square of wet and dry samples were prepared as previously described in order to carry out air permeability measurements. The sample is placed on the device as instructed by the manufacturer. An aspiration pump is used to generate a depression of 1210 Pa that sucks air through the sample layer. The device measures the volume of air flow at constant pressure drop across the orifices that contains the sample and measurement head. Finally the device generates a value of air permeability in the units of "l/ (m$^2$s)". For each material, in the dry and in the wet state, six samples are measured and the average value of air permeability calculated.

Results

The air permeability of the apertured swellable layers are given below:

| Swellable layer | Dry (l/m$^2$/s) | Wet (l/m$^2$/s) |
|---|---|---|
| Example 1 | 72 | zero |
| Example 2 | 116 | zero |
| Example 3 | 105 | 6 |

From the above results it can be clearly seen that the exemplified backsheet layer of the present invention have negligible air permeability after contact with liquid.

Water vapour permeability tests on individual backsheet swellable layers

The Vapour permeability test is utilised to quantify the vapour transmission properties of breathable backsheet layers.

Basic Principle of the Method:

The basic principle of the test is to quantify the extent of water vapour transmission of a material. The test method that is applied is based on a standardized textile industry applied test method and commonly referred to as the Upright cup test method. The test is performed in a stable temperature/humidity laboratory maintained at a temperature of 23 °C at 50% RH for a period of 24 hours.

Apparatus:

1) Sample cup (open area=0.00059 m2)
2) Syringe to introduce the distilled water into the completed sample cup.
3) Wax to seal the cup once sample has been arranged.
4) A circular punch to facilitate preparation of circular samples of diameter = 30 mm.
5) Laboratory of stable climatic conditions (23° C $\pm$ 0.5°C / RH = 50% $\pm$ 1 % RH)
6) Laboratory balance accurate to 4 decimal places.

Sample Preparation / Measurements:

The test may be performed on the individual swellable layer from which a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup.

The closure ring of the sample cup is then placed onto the sample and pushed down. This ensures that the excess material is held firmly in place and does not interfere with the measurement. A molten wax is then applied to the entire surface of the closure ring to ensure the whole upper part of the apparatus is closed to the environment. Distilled water (5 ± 0.25 ml) is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is 3 ±0.3 m/sec and confirmed via a wind velocity meter ("Anemo", supplied by Deuta SpA., Italy). The sample cup remains in the ventilated test field for a period of 24 hrs and is re-weighted once per hour for the first 6 hours and at the end of the 24 hrs period. During this period if the test sample is sufficiently breathable the liquid in the sample holder is able to diffuse out of the sample holder and into the laboratory environment. This results in a reduction in the weight of water in the sample holder that can be quantified on reweighing the complete sample cup following the 24 hrs period.

The vapour permeability value is determined as the weight loss divided by the open area of the sample holder and quoted per day.

$$\text{i.e. Vapour Permeability} = \text{Weight Loss (g)} / (0.00059 \text{ m}^2 / 24 \text{ hrs})$$

The water vapour permeability of wet samples is determined in the same way, by using samples that have been wetted as described herein above. The material is then sealed on the sample cup and the test started within 10 minutes. For each material, in the dry and in the wet state, six samples are measured and the average value of water vapour permeability calculated.

It should be noted that at the beginning of the water vapour permeability test with the wet samples, an unsteady state situation will initially be observed. This is due to the fact that the sample loses part of its humidity, i.e. the liquid added in order to produce a wet sample, into the surrounding environment. However this "unsteady" state is relatively short and, according to observations the humidity weight loss becomes linear (so that the sample is in equilibrium with the system). Hence, the only exchange of water vapour is the permeation of water through the sample of the vapour derived from the inside of the cup in not more than 1 hour. Consequently, it is necessary when recording the weights as herein described to only take into account the water vapour permeability measurements which demonstrate a progressive linear weight loss of the test cup versus time, within the 24 hrs time period.

Results

| Swellable layer examples | Water Vapour permeability g/(m$^2$.24hrs) | |
|---|---|---|
| | Dry | Wet |
| Example 1 | 638 | 481 |
| Example 2 | 777 | 378 |
| Example 3 | 767 | 558 |

From the above results, it can be concluded that the apertured swellable layers retain a substantial degree of water vapour permeability after contact with liquid, during which swellage and closure of the apertures occurs. This is believed to be linked to the considerable hydrophilicity of the tested swellable materials according to the present invention, which leads to a high diffusion of water vapour into and through the layers, even in the absence of physical apertures.

Swellage test

The ability of the material to swell was measured by determining the caliper of a test sample of a layer in the dry and wet state. This determines the swellage in the z direction.

A square sample of 100 mm size of the layer to be tested, is prepared for caliper (thickness) measurement in the dry and wet state. The samples calipers are measured by using a thickness measuring device App. 51D20, Type 02101, supplied by Lorentzen & Wettre, Sweden and following the instruction of the supplier. The thickness measuring device records the caliper of the sample pressed between the two measuring plates, when the exerted pressure reaches 20 g/cm$^2$. The average of three caliper readings of each sample is taken. Five samples for each material are tested and the value is the average value of these readings.

| Swellable film | Dry caliper (mm) | Wet caliper (mm) | % delta swellage z direction |
|:---:|:---:|:---:|:---:|
| Example 1 | 40 | 130 | 225 |
| Example 2 | 50 | 160 | 220 |
| Example 3 | 80 | 190 | 137 |

From the above values, it can be observed that the exemplified layers demonstrate a high degree of swelling such that the caliper of the wet samples is from 200% to 300% greater than the caliper of the corresponding dry samples, even after a liquid contact time with water of only 60 seconds. It is believed that this high rate of swelling accounts for the ability and effectiveness of the these materials in the backsheet layers of the present invention to rapidly close the apertures of layers and thereby reduce the air permeability.

Example 3 although still exhibiting a high degree of swellage does not swell to the same degree as Examples 1 and 2. It is believed that this behaviour may be related to the characteristics of the Polyvinyl alcohol itself and in particular to the high resistance of this polyvinyl alcohol layer to dissolve even at a water temperature of 80 °C. Such a high resistance to dissolution is thus also associated with a greater resistance to plasticisation by water at lower temperatures. Consequently, the polyvinyl alcohols will exhibit reduced swelling ability under the test conditions.

**Claims**

1. An absorbent article comprising a liquid pervious topsheet, a backsheet and an absorbent core, said core positioned between said topsheet and said backsheet, said backsheet being breathable, **characterized in that** said backsheet comprises at least one layer selected from a woven or an apertured film and said layer comprises from 10% to 100% by weight of a non soluble, liquid swellable material, and said layer has a swellage of at least 100 % in one direction as defined in the swellage test.

2. An absorbent article according to claim 1, wherein said swellable material is a polyvinyl alcohol .

3. An absorbent article according to claim 2, wherein said polyvinyl alcohol has a hydrolysis level of at least 85%, preferably at least 98%.

4. An absorbent article according to any one of the preceding claims, wherein said layer of said backsheet has an air permeability when dry of at least 50 l/(m$^2$s) and an air permeability when wet of less than 10 l/(m$^2$s) as defined in the air permeability test

5. An absorbent article according to any one of the preceding claims, wherein said layer of said backsheet, when dry has a water vapour permeability of at least 500 g/(m$^2$.24hrs), as defined in the vapour permeability test.

6. An absorbent article according to any one of the preceding claims, wherein said layer of said backsheet, when wet has a water vapour permeability of more than 250 g/(m$^2$.24hrs), as defined in the vapour permeability test.

7. An absorbent article according to any one of the preceding claims, wherein said layer is selected from a fibrous woven, a 2-Dimensional microporous apertured film, or a 2-Dimensional macroporous apertured film , wherein said layer has an average aperture diameter of from 5 micrometers to 600 micrometers.

8. An absorbent article according to any one of the preceding claims, wherein said backsheet comprises at least two layers, a first layer comprising said liquid swellable material and a second non liquid swellable layer comprising a fibrous woven, a fibrous nonwoven, a 2-Dimensional apertured porous film or a apertured formed film.

9. An absorbent article according to any one of the preceding claims, wherein said backsheet consists of three layers, a first layer comprising said non soluble, liquid swellable material, a second non liquid swellable layer comprising an apertured formed film and a third non liquid swellable layer comprising a fibrous nonwoven.

**Patentansprüche**

1. Absorbierender Artikel mit einer flüssigkeitsdurchlässigen Oberschicht, einer Unterschicht und einem absorbierenden Kern, wobei der Kern zwischen der Oberschicht und der Unterschicht positioniert ist, wobei die Unterschicht atmungsfähig ist, **dadurch gekennzeichnet, daß** die Unterschicht wenigstens eine Schicht umfaßt, ausgewählt aus einem Gewebe oder einem geöffneten Film, und die Schicht von 10 Gew.% bis 100 Gew.% eines nicht löslichen, in Flüssigkeit quellfähigen Materials umfaßt und eine Quellfähigkeit von wenigstens 100% in einer Richtung hat, wie dies in dem Quellfähigkeitstest definiert ist.

2.  Absorbierender Artikel nach Anspruch 1, in welchem das quellfähige Material ein Polyvinylalkohol ist.

3.  Absorbierender Artikel nach Anspruch 2, in welchem der Polyvinylalkohol einen Hydrolysegrad von wenigstens 85%, vorzugsweise wenigstens 98% hat.

4.  Absorbierender Artikel nach einem der vorstehenden Ansprüche, in welchem die Schicht der Unterschicht eine Luftdurchlässigkeit im trocknen Zustand von wenigstens 50 l/(m$^2$s) und eine Luftdurchlässigkeit im feuchten Zustand von weniger als 10 l/(m$^2$s) hat, wie dies im Luftdurchlässigkeitstest definiert ist.

5.  Absorbierender Artikel nach einem der vorstehenden Ansprüche, in welchem die Schicht der Unterschicht im trocknen Zustand eine Wasserdampfdurchlässigkeit von wenigstens 500 g/(m$^2$24h) hat, wie dies im Dampfdurchlässigkeitstest definiert ist.

6.  Absorbierender Artikel nach einem der vorstehenden Ansprüche, in welchem die Schicht der Unterschicht im nassen Zustand eine Wasserdampfdurchlässigkeit von mehr als 250 g/(m$^2$24h) hat, wie dies im dem Dampfdurchlässigkeitstest definiert ist.

7.  Absorbierender Artikel nach einem der vorstehenden Ansprüche, in welchem die Schicht ausgewählt ist aus einem faserigen Gewebe, einem zweidimensionalen, mikroporös geöffneten Film oder einem zweidimensionalen makroporös geöffneten Film, wobei die Schicht einen mittleren Öffnungsdurchmesser von 5 Mikrometer bis 600 Mikrometer hat.

8.  Absorbierender Artikel nach einem der vorstehenden Ansprüche, in welchem die Unterschicht wenigstens zwei Schichten umfaßt, wobei eine erste Schicht das in Flüssigkeit quellfähige Material umfaßt und eine zweite nicht in Flüssigkeit quellfähige Schicht ein faseriges Gewebe, einen faserigen Vliesstoff, einen zweidimensional geöffneten porösen Film oder einen offen geformten Film umfaßt.

9.  Absorbierender Artikel nach einem der vorstehenden Ansprüche, in welchem die Unterschicht aus drei Schichten besteht, einer ersten Schicht mit dem nicht löslichen, in Flüssigkeit quellfähigen Material, eine zweite nicht in Flüssigkeit quellfähige Schicht mit einem offen geformten Film und einer dritten nicht in Flüssigkeit quellfähigen Schicht mit einem faserigen Vliesstoff.

## Revendications

1.  Article absorbant comprenant une feuille de dessus perméable aux liquides, une feuille de fond et une âme absorbante, ladite âme étant placée entre ladite feuille de dessus et ladite feuille de fond, ladite feuille de fond pouvant respirer, **caractérisé en ce que** ladite feuille de fond comprend au moins une couche choisie entre un tissé ou un film perforé, et ladite couche comprend entre 10% et 100% en poids d'un matériau non soluble, gonflable de liquide, et a un gonflement au moins de 100% dans une direction, comme cela est défini dans l'essai de gonflement.

2.  Article absorbant selon la revendication 1, dans lequel ledit matériau gonflable est un alcool polyvinylique.

3.  Article absorbant selon la revendication 2, dans lequel ledit alcool polyvinylique a un niveau d'hydrolyse au moins de 85%, de préférence, au moins de 98%.

4.  Article absorbant selon l'une quelconque des revendications précédentes, dans lequel ladite couche de ladite feuille de fond a une perméabilité à l'air, lorsqu'elle est sèche, d'au moins 50 l/(m$^2$s), et une perméabilité à l'air, lorsqu'elle est humide, inférieure à 10 l/(m$^2$s), comme cela est défini dans l'essai de perméabilité à l'air.

5.  Article absorbant selon l'une quelconque des revendications précédentes, dans lequel ladite couche de ladite feuille de fond, lorsqu'elle est sèche, a une perméabilité à la vapeur d'eau d'au moins 500 g/(m$^2$.24h), comme cela

est défini dans l'essai de perméabilité à la vapeur.

6. Article absorbant selon l'une quelconque des revendications précédentes, dans lequel ladite couche de ladite feuille de fond, lorsqu'elle est humide, a une perméabilité à la vapeur d'eau supérieure à 250 g/(m$^2$.24h), comme cela est défini dans l'essai de perméabilité à la vapeur.

7. Article absorbant selon l'une quelconque des revendications précédentes, dans lequel ladite couche est choisie parmi un tissé fibreux, un film perforé microporeux à 2 dimensions ou un film perforé macroporeux à 2 dimensions, dans lequel ladite couche a un diamètre d'ouverture moyen compris entre 5 µm et 600 µm.

8. Article absorbant selon l'une quelconque des revendications précédentes, dans lequel ladite feuille de fond comprend au moins deux couches, une première couche comprenant ledit matériau gonflable de liquide et une deuxième couche non gonflable de liquide comprenant un tissé fibreux, un non-tissé fibreux, un film poreux perforé à 2 dimensions ou un film formé perforé.

9. Article absorbant selon l'une quelconque des revendications précédentes, dans lequel ladite feuille de fond est constituée de trois couches, une première couche comprenant ledit matériau gonflable de liquide, non soluble, une deuxième couche non gonflable de liquide comprenant un film formé perforé et une troisième couche non gonflable de liquide comprenant un non-tissé fibreux.